# EUROPEAN PATENT APPLICATION

(11) **EP 1 543 823 A1**
(43) Date of publication of application: **22.06.2005**
(21) Application number: 03293264.2
(22) Date of filing: 19.12.2003
(51) Int. Cl.: A61K 7/48

(54) **Gingko containing formulations for the improvement of skin radiance**

(71) Applicant: Johnson & Johnson Consumer France SAS, 92787 Issy les Moulineaux Cedex 9 (FR)
(72) Inventor: Courcoux, Laetitia, 27220 Garencieres (FR); Quiry, Nathalie, 27400 Hondouville (FR); Oddos, Thiery, 92190 Meudon (FR); Porracchia, Jean-Francois, Princeton, 08540 (US); Issachar, Nathalie, 75016 Paris (FR)
(74) Representative: Weber-Bruls, Dorothée, Dr.

(57) **Abstract**

This invention relates to topical formulations containing both feverfew extract and Gingko biloba extract and the use thereof to increase skin radiance.

## Description

### Field of the invention

This invention relates to topical formulations containing both feverfew extract and Gingko biloba extract and the use thereof to increase skin radiance.

### Background of the Invention

The appearance and condition of the skin may be degraded through external factors such as sunlight, exposure to wind and to cool and dry air, air pollutants, smoking, as well as internal factors such as dermatological diseases, age-related hormonal changes or the normal aging process. In particular upon aging, the skin becomes less elastic and develops fine lines and wrinkles. Other phenomena that develop upon aging comprise skin thinning, skin sagging, and age spots appear, and the skin loses its tone and natural radiance. Specifically the decrease of skin radiance is attributed to the accumulation of toxins in skin cells coming from cell metabolism created by external factors such as sunlight or pollution. This creates non-homogeneous color areas in the skin or dull skin. These toxins mainly comprise reactive oxygen species.

To counteract skin degradation and in particular to prevent and level the effects of skin-aging, consumers have increasingly sought new and/or improved cosmetic compositions and cosmetic methods for skin care. To meet consumer demand, cosmetic products have been developed for treating the effects of skin-aging, which products for example are based on active ingredients such as vitamin A or its derivatives, alpha-hydroxy acids, vitamin C or plant extracts.

Reduced skin tone and/or skin radiance is one of the elements in the skin-aging process. To counteract this, use has been made of pigments or makeup, which had a cosmetic (or exterior) effect, which is only temporary, but did not interfere with the root cause of the problem. The use of certain mineral components that enlarge the blood vessels was aimed at a more in depth approach of dealing with this problem.

WO-00/74699 discloses the use of compositions containing extracts of feverfew against inflammatory disorders. Particular extracts for this application are extracts that are substantially free of α-unsaturated γ-lactones.

US-20020182166 discloses a composition for regulating the firmness, tone or texture of skin, or for regulating wrinkles, or for the treatment of external aggression in skin containing a safe and effective amount of feverfew extract and a cosmetically-acceptable topical carrier, and the use thereof.

Tanacetum parthenium, a plant commonly known as feverfew, has been recognized for a long time as having significant medicinal properties when taken orally. Extracts of feverfew contain many components. Although not all components have been isolated and characterized, the known components of an extract of feverfew contain a significant number of biologically active components. To date, the chemical constituents of whole feverfew extract include, but are not limited to, apigenin-7-glucoside, apigenin-7- glucuronide, 1-β-hydroxyarbusculin, 6-hydroxykaempferol-3,7-4'- trimethylether (Tanetin), 6-hydroxykaempferol-3,7-dimethyl ether, 8-β- reynosin, 10-epicanin, ascorbic acid, beta-carotene, calcium, chromium, chrysanthemolide, chrysanthemomin, chrysarten-A, chrysarten-c, chrysoeriol-7-glucuronide, cobalt, cosmosiin, epoxyartemorin, luteolin-7-glucoside, luteolin-7-glucuronide, mangnoliolide, parthenolide, quercetagentin-3,7,3,-trimethylether, quercetagetin-3'7-dimethylether, reynosin, tanaparthin, tanaparthin-1α,4α-epoxide, tanaparthin- 1β,4β-epoxide, β-costunolide, 3-β-hydroxy-parthenolide, and 3,7,3'-trimethoxyquercetagetin.

Ginkgo biloba is a dioecious tree from the Far East, the leaves of which are used for certain medicinal properties. They contain constituents, such as aliphatic hydrocarbons and alcohols, polyphenols, such as luteolin or quercetol, or biflavones derived from amentaflavone, and more specific constituents: ginkgolic acid, and anacardic derivatives, terpenes derived from limonene or terpenes containing a tert-butyl group. They have been used in cosmetology for their protective effect against free radicals.

EP-A-0955995 relates to the use of at least two compounds chosen from components having an a) anti-radical, b) anti- inflammatory and c) anti-allergic activity for the preparation of a composition exhibiting at least two of the a), b) and c) activities intended for the treatment of sensitive and/or allergic skin, amongst which is Gingko biloba extract.

EP-A-0877619 relates to the use of at least one active ingredient capable of being obtained by extraction from Ginkgo biloba for the preparation of a composition with immunomodulatory activity and further relates to methods for the cosmetic treatment of sensitive skins.

It is an object of this invention to provide formulations that have a positive effect on skin radiance and improve complexion.

The formulations of the present invention containing Gingko biloba, which is an extract that has anti-free radical activity, and feverfew extract, which has antiinflammatory activity, have been found to have a beneficial effect on skin radiance.

### Summary of the invention

Thus in one aspect, the present invention concerns a cosmetic formulation containing feverfew extract and Gingko Biloba extract.

In a particular aspect there is provided a cosmetic formulation containing
(a) from 0.01% to 5% of feverfew extract; and
(b) from 0.005% to 1% of Gingko biloba extract.

In another aspect, the invention features the use of a cosmetic formulation as specified herein for maintaining or improving skin radiance.

### Detailed description of the invention

As used herein, all percentages are by weight unless otherwise specified. As used herein the term 'cosmetic' relates to applications on the skin in order to have skin-beneficial effects and is meant to relate to terms such as topical and skin care.

As used herein the term 'feverfew extract' is meant to comprise a blend of components isolated from a plant from the Chrysanthemum or Tanacetum genera (hereinafter referred to as Feverfew). Examples of Feverfew include, bur are not limited to, Chrysanthemum parthenium, Tanacetum parthenium, or Matricania parthenium.

Such components may be isolated from a part(s) of the plant (e.g., the arial part of the plant such as the stem, flower, and leaves) by physically removing a piece of such plant, such as grinding a leaf on the plant. Such components may also be isolated from the plant by using extraction procedures well known in the art (e.g., the use of organic solvents such as C₁-C₈ alcohols, C₁- C₈ alkyl polyols, C₁-C₈ alkyl ketones, C₁-C₈ alkyl ethers, acetic acid C₁-C ₈ alkyl esters, and chloroform, and/or inorganic solvents such as water, inorganic acids such as hydrochloric acid, and inorganic bases such as sodium hydroxide). In one embodiment, the feverfew extract contains only hydrophilic components (e.g., isolated by using a hydrophilic solvent, such as water or ethanol). In one embodiment, the Feverfew extract contains only hydrophobic components (e.g. isolated by using a hydrophobic solvent, such as chloroform). In one embodiment, the Feverfew extract contains both hydrophilic and hydrophobic components.

A particular feverfew extract is that described in WO-00/74699, which is feverfew extract substantially free of α-unsaturated γ-lactones. The term "substantially free of alpha-unsaturated gamma-lactones" refers to an extract of feverfew having a weight content of the α-unsaturated γ-lactones found in natural feverfew extracts of less than about 0.2% by weight. These α-unsaturated γ-lactones include but are not limited to parthenolide ([1αR -(1a R*, 4E,7a S*, 10a S*, 10b R*)]2,3,4,7,7a,8,10a,10b-octahydro-1a,5-dimethyl-8-4,5α-epoxy-6β-hydroxy- germacra-1(10),1 1(13)-dien-12-oic acid γ-lactone), 3-β-hydroxy-parthenolide, costunolide, 3-β-constunolide, artemorin, 8-α-hydroxy-estafiatin, chysanthemolide, magnoliolide, tanaparthin, tanaparthin-1α,4α-epoxide, tanaparthin-1β,4β-epoxide, chrysanthemonin, and other sesquiterpenes. Preferably, the feverfew extract has a weight content of below about 0.2. Preferably, the feverfew extract has a weight content of α-unsaturated γ-lactones below about 0.2% by weight. Preferably the α-unsaturated γ-lactone is parthenolide.

Methods for the manufacture of Feverfew extracts that are substantially free of parthenolide and other alpha-unsaturated gamma-lactones are disclosed in WO-00/74695.

Of interest are formulations with low content of parthenolide. What is meant by "low content of parthenolide" is that the composition comprises, by weight, less than 0.1%, preferably below 0.01 %, more preferably below 0.001 % or does not comprise any parthenolide.

Since the α-unsaturated γ-lactones cause some of the allergic reactions to extracts of feverfew, topical compositions made from α-unsaturated γ-lactone-deprived extracts are preferred because of their non-irritating/non-sensitizing properties.

The Feverfew extract may contain the following compounds: flavanoid/flavone compounds which include, but are not limited to, tanetin, 3,7,3'-trimethoxy quercetagetin, apigenin and its derivatives. When flavanoid/flavone compounds are present, they are present at a concentration of between about 0.001% to about 0.5% such as between about 0.005% and 0.2% based on the weight of the topical composition.

As used herein the term 'Gingko biloba extract' is meant to comprise a blend of components isolated from Gingko biloba. Various Ginkgo biloba extracts may be suitable for carrying out the invention; preferably, the terpene concentration in these extracts is less than about 7% w/w. Indeed, Ginkgo biloba has a complex chemical composition which comprises aliphatic alcohols and hydrocarbons, steroids, amino acids, polyphenols such as luteolin, quercetol and more specific biflavones derived from amentaflavone (bilobetin, ginkgetin); it also contains ginkgolic acid and sesquiterpene-chain- containing anacardic derivatives, terpenes derived from limonene and terpenes with tert-butyl groups (bilobalides and Ginkgolides).

Extracts appropriate for carrying out the invention may have a content of terpene derivatives with a value of less than about 3% w/w of dry matter, especially of less than 1% w/w and advantageously of less than 0.5% w/w. Preferably, chlorophyll, lipids, waxes and lectins are substantially removed from the extract used. By preference, the extract also substantially lacks substances such as ginkgolic acid, biflavonoids or free aglycones. The concentration of proanthocyanidins is preferably less than 5% w/w of the dry extract. Extracts that can be used according to the invention preferably have a concentration of flavone heterosides greater than 24% w/w of dry matter, more preferably greater than 28% w/w; it is advantageously between about 29 and 35% w/w. The flavone heterosides are compounds whose aglycone is a flavonol such as quercetin, kaempferol or isorhamnetin. These are mono-, tri- and especially diglucosides, of which the principal carbohydrate constituents are glucose and rhamnose. They are present in a substantial proportion in the form of coumarin esters of quercetin and of kaempferol glucorhamnoside. A Ginkgo extract which is particularly suitable for carrying out the invention has a water content of less than 3% w/w, a concentration of flavone heterosides of 32 ± 3% w/w, a Ginkgolic acid concentration of less than 10 ppm, a proanthocyanidin concentration of less than 5% w/w and a terpene concentration of less than 0.5% w/w. Particular extracts for use in this invention, as well as their preparation have been described in EP-A-0877619

The extracts may be dry extracts or may be liquid extracts, e.g. in aqueous media. Dry extracts are preferred.

The Fever few extract may be present a at a concentration which can be between 0,01 % and 5%, in particular between 0.05% and 2%, further in particular between 0.05 and 1%, e.g. at about 0.1%.

The Ginko biloba extract may be present at a concentration which can be between 0,005% and 1%, in particular between 0.01% and 0.05%.

In certain embodiments of the invention, the w/w ratio between the total amount of Feverfew extracts and the total amount of Gingko biloba extracts is in the range of from 1000 : 1 to 1 : 100; in particular from 100 : 1 to 1 : 10; more in particular from 10 : 1 to 1 : 1.

The formulations in accordance with the invention may additionally contain other plant extracts. Of particular interest are soy extracts, preferably the soy extracts disclosed in EP-A-1236465. The soy extracts can be present at a concentration, which may be between 0.01 % to 5 %, in particular between 0.1 % and 3 %, preferably between 0.5% and 2%, e.g. at a concentration of about 1 %.

The formulations may further contain vitamins such as Vitamin E or a derivative thereof such as tocopherol acetate. The Vitamin E may be present at a concentration which can be between 0.01 % to 1%, with an optimal at 0,3%.

The topical formulations of the present invention may comprise the Feverfew extract, the Gingko biloba extract and a cosmetically-acceptable topical carrier. In certain embodiments, the cosmetically-acceptable topical carrier is from about 50% to about 99.99%, by weight, of the composition (e.g., from about 80% to about 95%, by weight, of the composition.

The compositions may be made into a wide variety of product types that include, but are not limited to, lotions, creams, gels, sticks, sprays, shaving creams, ointments, cleansing liquid washes and solid bars, shampoos, pastes, powders, mousses, shaving creams, wipes, patches, nail lacquers, wound dressing and adhesive bandages, hydrogels, films and make- up such as foundations, mascaras, and lipsticks. These product types may comprise several types of cosmetically-acceptable topical carriers including, but not limited to, solutions, emulsions (e.g., microemulsions and nanoemulsions), gels, solids and liposomes. The following are non- limitative examples of such topical carriers. Other topical carriers can be formulated by those of ordinary skill in the art.

The topical formulations of the present invention can be formulated as solutions. Solutions typically include an aqueous solvent (e.g., from about 50% to about 99.99% or from about 90% to about 99% of a cosmetically acceptable aqueous solvent).

The topical formulations of the invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. The International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7^{th} Edition, 1997) (hereinafter "ICI Handbook") contains numerous examples of suitable materials.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in the ICI Handbook pp. 1693-1697.

The topical formulations useful in the present invention are formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, U.S. Pat. Nos. 3,755,560, 4, 421,769, McCutcheon's Detergents and Emulsifiers, North American Edition, pp. 317-324 (1986), and the ICI Handbook, pp.1673-1686.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e. g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The topical formulations of this invention can also be formulated as a gel (e.g., an aqueous gel using a suitable gelling agent(s). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprises between about 0.1% and 5%, by weight, of such gelling agents.

The topical formulations of the present invention can also be formulated into a solid formulation (e.g., a wax-based stick, soap bar formulation, powder, or a wipe containing powder).

Liposomal formulations are also useful formulations of the subject invention. Examples of liposomes are unilamellar, multilamellar, and oligolamellar liposomes, which may or may not contain phospholipids. Such compositions can be prepared by first combining hesperetin with a phospholipid, such as dipalmitoylphosphatidyl choline, cholesterol and water according to art-known methods.. Epidermal lipids of suitable composition for forming liposomes may be substituted for the phospholipid. The liposome preparation may then be incorporated into one of the above carriers (e.g., a gel or an oil-in- water emulsion) in order to produce the liposomal formulation.

The topical formulations useful in the subject invention may contain, in addition to the aforementioned components, a wide variety of additional oil-soluble materials and/or water-soluble materials conventionally used in formulations for use on skin, hair, and nails at their art-established levels.

In one embodiment, the topical formulation comprises one or more cosmetically active agents. What is meant by a "cosmetically active agent" is a compound that has a cosmetic or therapeutic effect on the skin, hair, or nails, e.g., lightening agents, darkening agents such as self-tanning agents, anti- acne agents, shine control agents, anti-microbial agents, anti- inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

Examples of cosmetically active agents are hydroxy acids, benzoyl peroxide, sulfur resorcinol, ascorbic acid, D-panthenol, hydroquinone, octyl methoxycinnamate, titanium dioxide, octyl salicylate, homosalate, avobenzone, polyphenolics, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides such as those disclosed in WO-00/15188, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera and soy, and derivatives and mixtures thereof. The cosmetically active agent will typically be present in the formulation of the invention in an amount of from about 0.001% to about 20% by weight of the formulation, e.g., about 0.01% to about 10% such as about 0.1% to about 5%.

Examples of vitamins include, but are not limited to, vitamin A, vitamin Bs such as vitamin B3, vitamin B5, and vitamin B 12, vitamin C, vitamin K, and vitamin E and derivatives thereof.

Examples of hydroxy acids include, but are not limited, to glycolic acid, lactic acid, malic acid, salicylic acid, citric acid, and tartaric acid (see, e.g., EP-A-273,202).

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the formulations of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the formulations of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the ICI Handbook.

Various other materials may also be present in the formulations useful in the subject invention. These include humectants, proteins and polypeptides, preservatives and an alkaline agent. Examples of such agents are disclosed in the ICI Handbook, pp. 1650-1667. The formulations of the present invention may also comprise chelating agents (e.g., EDTA) and preservatives (e.g., parabens). Examples of suitable preservatives and chelating agents are listed in pp. 1626 and 1654-55 of the ICI Handbook. In addition, the topical formulations useful herein can contain conventional cosmetic adjuvants, such as dyes, opacifiers (e.g., titanium dioxide), pigments and fragrances.

The formulations of the present invention may be prepared using a mineral water. In one embodiment, the mineral water has a mineralization of at least about 200 mg/l (e.g. from about 300 mg/l to about 1000 mg/l). In one embodiment, the mineral water comprises at least about 10 mg/l of calcium and/or at least about 5 mg/l of magnesium.

The formulations of the invention are for improving skin radiance but may also be used for regulating one ore more skin-aging factors such as the firmness, tone or texture of skin, and regulating wrinkles in skin.
As used herein, "regulating the firmness of skin" means the enhancing of the firmness or elasticity of the skin, preventing the loss of firmness or elasticity of skin, or preventing or treating sagging, lax and loose skin. The loss of skin elasticity or firmness may be a result of a number of factors, including but not limited to aging, environmental damage, or the result of an application of a cosmetic to the skin.
As used herein, "regulating the tone of skin" means the lightening and/or darkening of the skin (e.g., lightening pigmented lesions or darkening skin sallowness).

As used herein, "regulating the texture of skin" means the smoothing of the surface of the skin to remove either bumps or crevasses on the skin surface.

As used herein, "regulating wrinkles in skin" means preventing, retarding, arresting or reversing the process of wrinkle and fine line formation in skin.
As used herein, "treatment of external aggressions in skin" means the reduction or prevention of the damage from external aggressions in skin. Examples of external aggressions include, but are not limited to, damage to the skin from the use of cleansers (e.g., topical cleansers containing surfactants), make-up, shaving as well as environmental damage such as from UV light (e.g., sun-damage from sunlight or damage from non-natural sources such as UV lamps and solar simulators), ozone, exhaust, pollution, chlorine and chlorine containing compounds and cigarette smoke. Effects of external aggressions on the skin include, but are not limited to, oxidative and/or nitrosative damage to and modifications on lipids, carbohydrates, peptides, proteins, nucleic acids, and vitamins. Effects of external aggressions on the skin also include, but are not limited to, loss of cell viability, loss or alteration of cell functions, and changes in gene and/or protein expression.

As used herein, "safe and effective amount" means an amount of compound or composition (e.g., the Feverfew extract) sufficient to significantly induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of concurrent therapy, the specific compound or composition employed, the particular cosmetically-acceptable topical carrier utilized, and like factors.

It furthermore has been found that the combined use of Feverfew extract in combination with Ginkgo biloba enhances the natural skin-detoxification mechanism and formulations containing this combination can be used for this purpose. Skin radiance is improved by applying this combination to the skin resulting in protecting and detoxifying effects helping better the skin to counteract toxins. The invention also relates to the treatment of external aggressions in skin including by using a cosmetical formulation as defined herein.

The invention is further illustrated by the following examples.

### Examples

### Example 1 :

An aqueous phase is made starting from the required amount of water and adding under stirring all water-soluble components. Separately an oil phase is made by mixing all oil components. Then the oily phase is added to the aqueous phase while stirring at increased temperature (75 - 80 °C). The whole is allowed to stir for a while to allow forming a stable emulsion. The whole is allowed to cool to ambient temperature whereupon the further components (butylene glycol, cyclopentasiloxane, dimethicone, Gingko extract, NaOH, perfume) are added under stirring.
The Gingko extract used in this and the following example is extract as prepared in the examples of EP-A-977619.
The feverfew extract used in this and the following example is extract substantially free of α-unsaturated γ-lactones as described in WO-00/74699.

| **INCI Name** | **% (w/w)** |
|---|---|
| Aqua | 74.2306 |
| Disodium EDTA | 0.02 |
| Panthenol 75%/Aqua 25% | 0.70 |
| Glycerin | 3.00 |
| C 10-30 Alkyl Acrylate Crosspolymer | 0.20 |
| Chrysanthemum Parthenium | 0.10 |
| Cetyl alcohol | 0.80 |
| Isononyl Isononanoate | 4.00 |
| Tocopheryl acetate | 0.60 |
| Isopropylparaben 40%/isobutylparaben 30%/ Butylparaben 30% | 0.80 |
| Glyceryl Stearate 50%/ PEG-100 Stearate 50% | 1.80 |
| Butyrospermum Parkii | 2.00 |
| Hexyldecyl stearate | 1.00 |
| Ceteth-20 5%/Cetyl Alcohol 40%/Glyceryl Stearate 32.5%/PEG-75 Stearate 17.5%/Steareth-20.5% | 1.00 |
| C13-14 isoparaffin 15-20%/Laureth-7 3-8%/Polyacrylamide 35-45%/Aqua 47-27% | 1.00 |
| Butylene glycol | 3.50 |
| Cyclopentasiloxane | 4.00 |
| Dimethicone | 1.00 |
| Gingko Biloba | 0.05 |
| Sodium Hydroxide | 0.0494 |
| Parfum | 0.15 |
| Total | 100.00 |

### Example 2 :

This formulation is made following the same procedure as described in Example 1.

| **INCI Name** | **% (w/w)** |
|---|---|
| Aqua | 77.04 |
| Disodium EDTA | 0.006 |
| Panthenol 75%/Aqua 25% | 0.70 |
| Glycerin | 5.00 |
| Aqua 46%/DMDM Hydantoin 54% | 0.26 |
| C10-30 Alkyl Acrylate Crosspolymer | 0.10 |
| Chrysanthemum Parthenium | 0.10 |
| Sodium Hydroxide 20%/Aqua 80% | 0.06 |
| Cetyl alcohol | 3.50 |
| Caprilic/ Capric Triglyceride | 2.00 |
| Hydrogenated Palm Glycerides Citrate 30-40%/Tocopherol 50-70% | 0.6 |
| Iodopropynyl Butylcarbamate 10%/PEG-4 10%/PEG-4 Laurate 40%/PEG-4 Dilaurate | 0.10 |
| Glyceryl Stearate 50%/ PEG-100 Stearate 50% | 3.00 |
| Butyrospermum Parkii | 0.50 |
| Sodium acrylate/Sodium Acryloyldimethyl Taurate Copolymer 25%/ Isohexadecane 20%/ Polysorbate 80 7%/ Aqua 48% | 0.80 |
| Cyclopentasiloxane | 4.50 |
| Dimethicone | 1.50 |
| Gingko Biloba | 0.01 |
| Propylene Glycol | 0.10 |
| Disodium EDTA | 2.00 |
| Parfum | 0.10 |
| Total | 100.00 |

## Claims

1. A cosmetic formulation containing feverfew extract and Gingko Biloba extract.

2. A cosmetic formulation according to claim 1 containing
(a) from 0.01% to 5% of feverfew extract; and
(b) from 0.005% to 1% of Gingko biloba extract.

3. A cosmetic formulation according to claims 1 - 2, wherein the w/w ratio between the total amount of Feverfew extracts and the total amount of Gingko biloba extracts is in the range of from 1000 : 1 to 1 : 100; in particular from 100 : 1 to 1 : 10; more in particular from 10 : 1 to 1 : 1.

4. A cosmetic formulation according to claims 1 - 3, wherein the feverfew extract substantially free of α-unsaturated γ-lactone

5. A cosmetic formulation according to claims 1 - 4 wherein the Gingko biloba extract has a content of terpene derivatives with a value of less than about 3% w/w of dry matter, especially of less than 1% w/w and advantageously of less than 0.5% w/w.

6. A cosmetic formulation according to claim 5 wherein in the Gingko biloba extract the chlorophyll, lipids, waxes and lectins are substantially removed from the extract; the extract also substantially lacks substances such as ginkgolic acid, biflavonoids or free aglycones; and the concentration of proanthocyanidins is preferably less than 5% w/w of the dry extract.

7. The use of a cosmetic formulation as claimed in claims 1 - 6 for maintaining or improving skin radiance.
